(19) Europäisches Patentamt
European Patent Office
Office européen des brevets

(11) Veröffentlichungsnummer: 0 167 759
A1

(12) # EUROPÄISCHE PATENTANMELDUNG

(21) Anmeldenummer: 85106150.7

(22) Anmeldetag: 20.05.85

(51) Int. Cl.[4]: C 07 C 69/03
C 07 F 7/18

(30) Priorität: 01.06.84 DE 3420510
30.10.84 DE 3439598

(43) Veröffentlichungstag der Anmeldung:
15.01.86 Patentblatt 86/3

(84) Benannte Vertragsstaaten:
BE CH DE FR GB IT LI SE

(71) Anmelder: BAYER AG
Konzernverwaltung RP Patentabteilung
D-5090 Leverkusen 1 Bayerwerk(DE)

(72) Erfinder: Schneider, Manfred, Prof. Dr.
Triebelsheider Weg 37
D-5600 Wuppertal 1(DE)

(72) Erfinder: Laumen, Kurt
Ohlerhof 30
D-4050 Mönchengladbach(DE)

(54) **Verfahren zur Herstellung von (1S, 4R)-4-Hydroxy-2-cyclopentenyl-estern.**

(57) (1*S*, 4*R*)-4-Hydroxy-2-cyclopentenylester werden in hoher optischer Reinheit erhalten, wenn man meso-cis-1,4-Diacyl-2-cyclopentene in Gegenwart von Schweineleberesterase hydrolysiert. Verschiedene neue Derivate dieser Verbingungen wurden hergestellt. Sie dienen als Zwischenprodukte für die Synthese von Arzneimitteln.

EP 0 167 759 A1

Croydon Printing Company Ltd

0167759

BAYER AKTIENGESELLSCHAFT
Konzernverwaltung RP
Patentabteilung

5090 Leverkusen, Bayerwer
Si/ja-c

# Verfahren zur Herstellung von (1S, 4R)-4-Hydroxy-2-cyclopentenylestern

Es wurde gefunden, daß man (1S, 4R)-4-Hydroxy-2-cyclopentenylester (II) in hoher optischer Reinheit erhält, wenn man meso-cis-1,4-Diacyl-2-cyclopentene (I) in Gegenwart von Enzymen hydrolysiert.

Dabei bedeutet in der Formel (I) Acyl einen aliphatischen Acylrest mit 1-18, vorzugsweise 1-6 Kohlenstoffatomen. Dabei ist insbesondere an den Acetyl- und den Propionylrest gedacht. Auch der 3-Phenylpropionylrest sei hier genannt.

Als Enzyme kommt vor allem Schweineleberesterase (P.L.E., E.C.3.1.1.1) in Frage. Weiterhin sind geeignet:
α-Chymotrypsin (E.C.3.4.21.1)
Acetylesterase (E.C.3.1.1.6)
Acetylesterase (Bac. subtilis)
Saccharomyces cerevisiae (Bäckerhefe)
Lipase (Candida cylindracea) (E.C.3.1.1.3)
Lipase (Rhizopus Sp.)
Bemerkenswerterweise erhält man bei der Verwendung der beiden letztgenannten Enzyme Produkte mit der umgekehrten absoluten Konfiguration.

Die erfindungsgemäß verwendeten Enzyme können sowohl in löslicher Form als auch als immobilisierte Enzyme, z. B. an Br-CN aktivierter Sepharose oder Oxiranacrylperlen, eingesetzt werden.

Insbesondere können nach dem erfindungsgemäßen Verfahren (1S, 4R)-4-Hydroxypentenylacetat (II; Ac = Acetyl) und (1S, 4R)-4-Hydroxypentenylpropionat (II; Ac = Propionyl) hergestellt werden. Die Ausgangsprodukte (I) sind in großen Mengen aus billigen Startmaterialien zugänglich [C. Kaneko, A. Sugimoto, S. Tanaka, Synthesis 1974, 376].

Die Verbindungen II, insbesondere II; R = Acetyl, und II; R = Propionyl, sind wertvolle Ausgangsprodukte für die Synthese von zahlreichen Cyclopentanoid-Naturprodukten, z. B. Brefeldin A, Sesquiterpene, Prostaglandine [vgl. M. Nara, S. Terashina, S. Yamada, Tetrahedron 36 (1980), 3161].

Durch selektive Manipulation der funktionellen Gruppen sind die Verbindungen II in die anderen Enantiomeren (1R, 4S) überführbar.

Die Verbindungen II besitzen vielseitige Reaktionsmöglichkeiten.

Es hat sich nämlich gezeigt, daß die Behandlung der Verbindungen II mit 2,3-Dihydropyran/p-TsOH (-)-(1S, 4R)-4-Tetrahydropyranoxy-2-cyclopentenylester (III) liefert. Diese gehen bei der Hydrolyse in Gegenwart von Schweineleberesterase in (+)-(1S, 4R)-4-Tetrahydropyranoxy-2-cyclopentanol (IV) über, welches ebenfalls in hoher optischer Reinheit anfällt.

Weiterhin können die Verbindungen II in Trialkylsilyloxyderivate (IX) in denen der Alkylrest 1-5 Kohlenstoffe enthält, bzw. das Tribenzylsilyloxyderivat übergeführt werden. Beispiele für die Silyloxygruppen solcher Silylether werden durch die nachstehenden Formeln veranschaulicht:

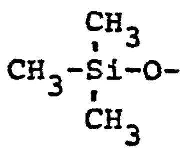 ,

Trimethylsilyl-

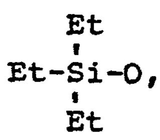

Triethylsilyl,

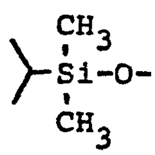 ,

Isopropyldimethylsilyl

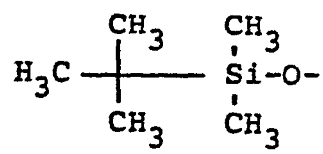

t-Butyldimethylsilyl-

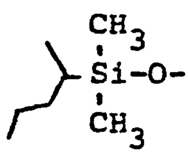

2-Methylbutyldimethylsilyl-

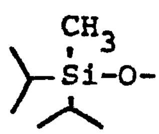

Diisopropylmethylsilyl

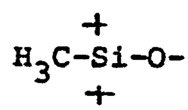

Di-t-butylmethylsilyl-

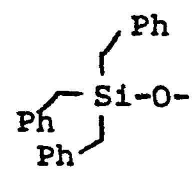

Tribenzylsilyl-

Diese Verbindungen eignen sich besonders gut als Zwischenprodukte für Synthese von Cyclopentanoid-Naturprodukten. Insbesondere seien genannt die 4-Trimethylsilyloxy-2-cyclopentenylester und die 4-tert.-Butyl-dimethylsilyloxy-2-cyclopentenylester.

Sowohl die Verbindungen II als auch IV lassen sich nach bekannten Methoden oxidieren zu (S)-4-Oxo-2-cyclopentenylestern (V) bzw. (R)-4-Tetrahydropyranoxy-2-cyclopentenon-(1) (VI).

Unterwirft man die Verbindungen II oder IV der Claisen-Umlagerung (Triethylorthoacetat/Hydrochinon; 160°C) so erhält man die Lactone VII bzw. ent-VII, die wertvolle Zwischenprodukte für die Synthese von Prostaglandinen sind. Durch dreistufige Verfahren über die "Prins"-Reaktion können VII und ent-VII in das "Corey-Lacton" (VIII) bzw. sein unnatürliches Enantiomer ent-VIII überführt werden.

Das erfindungsgemäße Verfahren erlaubt die Herstellung der Verbindungen II, insbesondere von II; R=Acetyl, in ausgezeichneten chemischen Ausbeuten und in optischen Anfangsreinheiten, die denjenigen bekannter Mehrstufenverfahren entsprechen [K. Ogura, M. Yamaschita, Tetrahedron Letters 1976, 759]. Im Gegensatz zu umständlichen Methoden der Diastereomerentrennung [M. Gill, R. W. Richards, Tetahedron Letters 1979, 1539] oder zur ebenso möglichen Anwendung des "meso-Tricks" [M. Nara, S. Terashima, S. Yamada, Tetahedron 36 (1980), 3161] liefert die enantioselektive Hydrolyse von I; R=Acetyl, nach dem erfindungsgemäßen Verfahren fast ausschließlich das einzige Enantiomer II, R=Acetyl.

Durch einfache Umkristallisation erhält man diese Verbindung optisch rein.

Das nachstehende Reaktionsschema erläutert die erfindungsgemäße Umsetzung I→II sowie die Weiterverarbeitung der erhaltenen Verbindungen II.

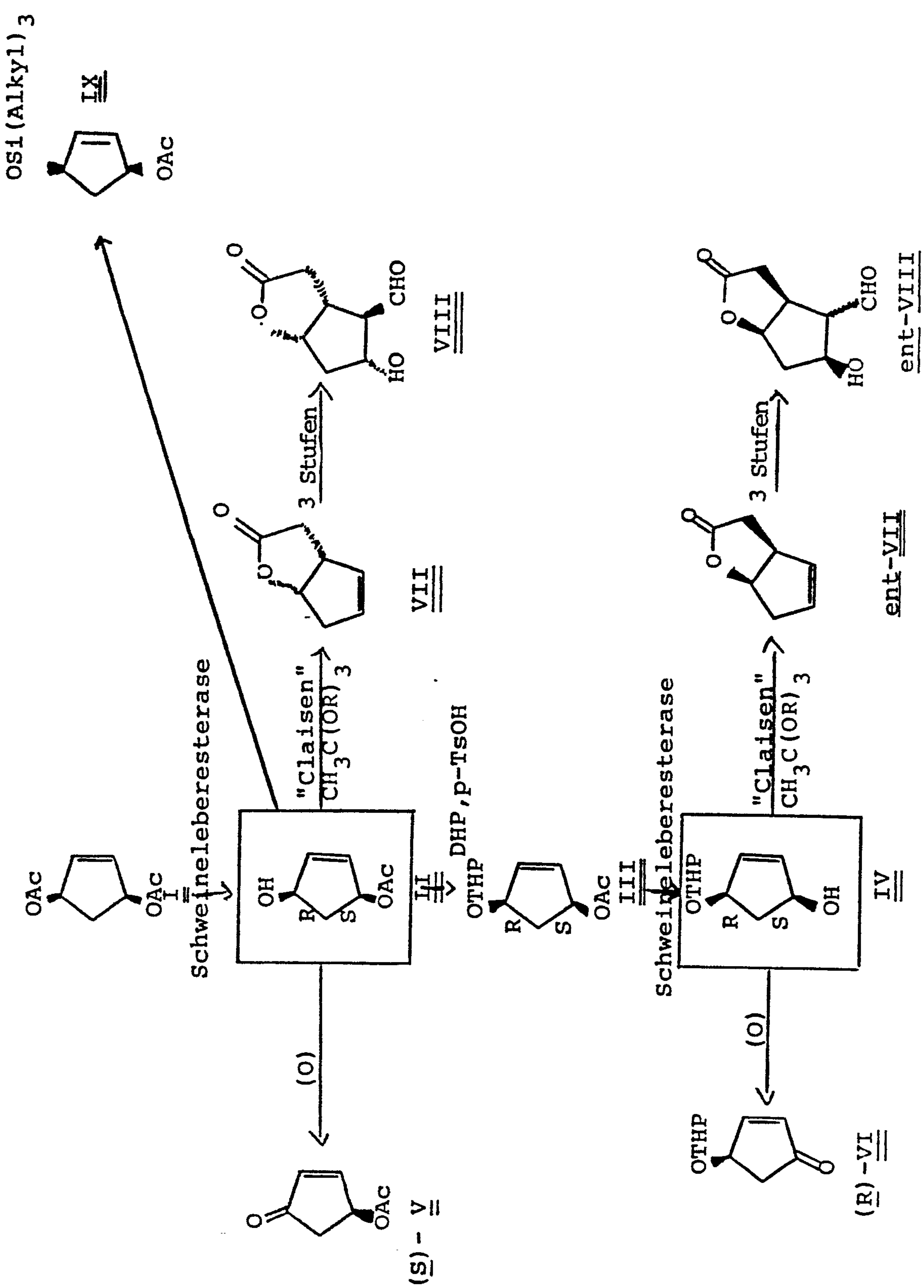
Schweineleberesterase
I
II
DHP, p-TsOH
III
Schweineleberesterase
IV
(O)
(S) - V
(O)
(R) -VI
"Claisen"
$CH_3C(OR)_3$
VII
3 Stufen
VIII
"Claisen"
$CH_3C(OR)_3$
ent-VII
3 Stufen
ent-VIII
IX

## Beispiel 1

12.88 g (70 mMol) meso-cis-1,4-Diacetoxy-2-cylopenten (I; R=Acetyl) werden in 140 ml 0,1 M Phosphatpuffer (pH 7) bei 32°C suspendiert und mit 1000 Einheiten löslicher Schweineleberesterase (P.L.E., E.C.3.1.1.1; 10 mg, Standard Bu-O-Ac) behandelt.

Die beginnende Verseifung wird durch schnellen Abfall des pH-Wertes angezeigt. Diesen hält man durch ständige Zugabe von 1 N NaOH-Lösung aus einer Autobürette konstant auf 7.

Nach Zugabe von 75 ml (1,04 äquiv.) NaOH wird die Mischung mit Ether extrahiert.

Bei der fraktionierten Destillation erhält man 8,6 g (86 % der Theorie) analytisch reines (-)-(1S, 4R)-4-Hydroxy-2-cyclopentenylacetat (II; R=Acetyl) vom $Kp_{0,2}$ 82°C und F. 32-33°C. Die Enantiomerenreinheit wurde mittels $^1$HNMR [250 MHz - $^1$H-NMR ($CDCl_3$)] $\delta$ = 1,62 (1H, dt, J = 4,15 Hz), 2,03 (3H, s, $CH_3$), 2,50 (1H, bd, J = 6,5 Hz, OH), 2,79 (1H, dt, J = 7,15 Hz), 4,71 (1H, m), 5,50 (1H, m), 6,05 (2H, AB, $J_{AB}$ = 7,5 Hz), und Verschiebunsreagenzien, wie Eu $(TFC)_3$, sowie durch gaschromatographische Analyse der (-)-$\alpha$-Methoxy-$\alpha$-trifluormethylphenylessigsäureester ermittelt. Das Enantiomerenverhältnis ist 83 : 17. (66 % e.e.). Der Drehwert beträgt $[\alpha]_D^{20}$ - 49.7 ° (c 0,86, $CHCl_3$).

Durch Umkristallisation aus Petrolether/Ether (9 : 1, - 15 °C) erhält man optisch reines /( > 95 % e.e.) Enantiomerenverhältnis 98 : 2 (Nachweisgrenze NMR, Cg)/ II; R = Acetyl, (63 % d. Th.). F. 49 bis 50 °C. $[\alpha]_D^{20}$ - 68.1 °. (c 0,27, $CHCl_3$).

Die entsprechende Umsetzung von meso-cis-1,4-Dipropionoxy-2-cyclopenten (I; R = Propionyl)mit dem gleichen Enzym liefert in einer Ausbeute von 87 % der Theorie (-)-(1S, 4R)-4-Hydroxy-2-cyclopentenylpropionat (II; Ac = Propionyl) vom Sdp. 74 °C / 0.2 torr.
Der Drehwert beträgt $[\alpha]_D^{20}$ - 38 ° (c 0,437, $CHCl_3$)
Das Enantiomerenverhältnis is 83 : 17 (66 % e.e.).
Verwendet man beim Substrat I; Ac = Acetyl, andere Enzyme, so erhält man folgende Ergebnisse:

| Enzym | abs. Konfiguration | chemische Ausbeute(%) | R : S (% e.e.) |
|---|---|---|---|
| α-Chymotrypsin (E.C. 3.4.21.1) | R | 73 | 71 : 29 (42) |
| Acetylesterase (E.C. 3.1.1.6) | R | 79 | 52 : 48 (4) |
| Acetylesterase (Bac. subtilis, ganzer Organismus) | R | 93 | 53 : 47 (6) |
| Saccharomyces cerevisiae (Bäckerhefe) | R | 87 | 87 : 13 (74) |
| Lipase (Candida cylindracea) (E.C. 3.1.1.3) | S | 82 | 25 : 75 (50) |
| Lipase /Rhizopus Sp.) | S | 83 | 17 : 83 (66) |

Die Umsetzung des Substrates I; Ac = Propionyl, mit Lipase (Candida cylindracea) (E.C.3.1.1.3) liefert II, Ac = Propionyl, mit der absoluten S-Konfiguration in einer chemischen Ausbeute von 60 %, R : S = 46 : 54 (8 % e.e.).

II; R = Acetyl, liefert bei der Umsetzung mit Tetrahydropyran und p-TsOH, 95 % der Theorie, (-)-(1S, 4R)-4-Tetrahydropyranoxy-2-cyclopentenylacetat (III, R = Acetyl); $[\alpha]_D^{20}$ - 8 ° (c 1,65, $CHCl_3$); dieses geht bei der Behandlung mit Schweineleberesterase unter milden Bedingungen bei pH 7 über in (+)-(1S 4R)-4-Tetrahydropyranoxy-2-cyclopentanol (IV). 95 % der Theorie, 1:1-Gemisch der Diastereomeren; $[\alpha]_D^{20}$ 21.5 ° (c 3,12, $CHCl_3$).

Beispiel 2

3,68 g (20 mMol) meso-cis-1,4-Diacetoxy-2-cyclopenten (I; R = Acetyl) werden in 50 ml 0,1 M Phosphatpuffer (pH 7) bei 32°C suspendiert und mit 20 ml einer Suspension der an Oxiranacrylperlen immobilisierten Schweineleberesterase versetzt.

Die beginnende Verseifung wird durch schnellen Abfall des pH-Wertes angezeigt. Diesen hält man durch ständige Zugabe von 1 N NaOH-Lösung aus einer Autobürette konstant auf 7. Nach Zugabe von 21 ml (1,05 eqm.) NaOH wird das immobilisierte Enzym durch Filtration (G3-Fritte) entfernt. Die verbleibende Lösung wird mit Ether extrahiert und das Produkt fraktioniert destilliert.

Man erhält 2.41 g (85 % der Theorie) analytisches reines II; R = Acetyl. $[\alpha]_D^{20}$ - 45.8°, c = 1,653, $CHCl_3$.

Beispiel 3

5-(S)-Acetoxy-3-(R)-trimethylsilyloxy-cyclopent-1-en (IX, Alkyl = Methyl, Ac = Acetyl).

1,113 g (7,84 mmol) (1S, 4R)-4-Hydroxy-2-cyclopentenyl-acetat (II, Ac = Acetyl) werden in 5 ml abs. Pyridin unter Zugabe von 0,1 g 4-(N,N-Dimethylamino)-pyridin und 20 ml $CCl_4$ gelöst. Unter Rühren tropft man 1,629 g (15 mmol) $(CH_3)_3SiCl$ zu und rührt 5 Minuten bei Raumtemperatur. Anschließend gibt man 20 ml gesättigte $NaHCO_3$-Lösung zu, schüttelt mit 50 ml Ether aus, trocknet die organische Phase über $Na_2SO_4$, verdampft das Lösungsmittel im Vakuum und destilliert den Rückstand nach Zugabe einer Spur Natriumacetat im Vakuum.

Ausbeute: 1,095 g (65 % der Theorie), einer farblosen Flüssigkeit vom Siedepunkt 51°C/0,2 Torr.

$[\alpha]_D^{20}$ = -3,6° c = 4,475 ($CHCl_3$)

In analoger Weise werden die nachstehenden Verbindungen der allgemeinen Formel

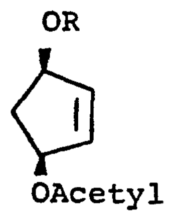

hergestellt:

| R | Siedepunkt | $[\alpha]_D^{20}$, $CHCl_3$ | c |
|---|---|---|---|
| Dimethyl-iso-propylsilyl | 76°C/0,2 torr | -3,4° | 3.169 |
| Dimethyl-2-pentyl-silyl | 107°C/0,2 torr | -2,9° | 4.201 |
| Diisopropyl-methylsilyl | 127°C/0,2 torr | -2,5° | 3.870 |
| Triethylsilyl | 121°C/0,2 torr | -3,1° | 3.253 |
| Triisopropyl-silyl | 168°C/0,05 torr | -2,0° | 4.139 |
| Dimethyl-tert.-butylsilyl | | -2,3° | 4.722 |
| Di-tert.-butyl-methylsilyl | | -1,7° | 4.538 |
| Tribenzylsilyl | | +0,7° | 3.225 |

Patentansprüche:

1. Verfahren zur Herstellung von (1S, 4R)-4-Hydroxy-2-cyclopentenylestern der Formel (II)

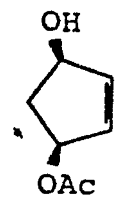

(II)

in der Ac für einen aliphatischen Acylrest mit 1-18 Kohlenstoffatomen steht,

dadurch gekennzeichnet, daß man

meso-cis-1,4-Diacyl-2-cyclopentene der Formel (I)

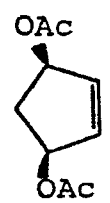

(I)

mit Enzymen behandelt.

2. Verfahren nach Anspruch 1, dadurch gekennzeichnet, daß Ac für einen aliphatischen Acylrest mit 1-6, vorzugsweise 1 bis 2 Kohlenstoffatomen steht.

3. Verfahren zur Herstellung von (1S, 4R)-4-Hydroxy-2-cyclopentenylacetat, dadurch gekennzeichnet, daß man meso-cis- 1,4-Diacetoxy-2-cyclopenten mit Schweineleberesterase behandelt.

4. Verfahren nach Anspruch 1 und 2, dadurch gekennzeichnet, daß man die Enzyme in immobilisierter Form einsetzt.

5. (1S, 4R)-4-Hydroxy-2-cyclopentenylacetat in einer optischen Reinheit von mindestens 95%.

6. 4-Trialkylsilyloxy-2-cyclopentenylester der Formel (IX)

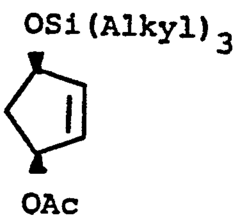

(IX)

in der der Alkylrest 1-5 Kohlenstoffatomen enthält und Ac die oben angegebene Bedeutung hat.

7. 4-Trimethylsilyloxy-2-cyclopentenylacetat.

8. 4-tert.-Butyldimethylsilyloxy-2-cyclopentenylacetat.

9. 4-Tribenzylsilyloxy-2-cyclopentenylacetat.

10. Verwendung von 4-Trialkylsilyloxy-2-cyclopentenylestern der Formel IX in Anspruch 6 und/oder von 4-Tribenzylsilyloxy-2-cyclopentenylacetat als Zwischenprodukte bei der Synthese von Cyclopentanoid-Verbindungen.

Europäisches Patentamt

# EUROPÄISCHER RECHERCHENBERICHT

0167759
Nummer der Anmeldung

EP 85 10 6150

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| | | | C 07 C 69/03<br>C 07 F 7/18 |
| X | FR-A-2 295 933 (TEIJIN)<br>* Patentansprüche; Beispiele 9,11 * | 1-3 | |
| | --- | | |
| X | FR-A-2 324 608 (TEIJIN)<br>* Seite 26, Beispiel 14 * | 8,10 | |
| | --- | | |
| A | TETRAHEDRON, Band 32, 1976, Seiten 1893-1898, Pergamon Press, GB; S. MIURA et al.: "Prostaglandin chemistry-IV. Microbiological kinetic resolution and asymmetric hydrolysis of 3,5-diacetoxycyclopent-1-ene" | 1,2 | |
| | --- | | RECHERCHIERTE SACHGEBIETE (Int. Cl 4) |
| A | CHEMICAL ABSTRACTS, Band 88, Nr. 25, 19. Juni 1978, Seite 557, Nr. 188167d, Columbus, Ohio, US; & JP - A - 78 05 140 (TAKANO SEIICHI; OGASAHARA, KUNIO; TANIGAWA, KEIZO) 18.01.1978 | 1,2 | C 07 C 69/00<br>C 07 F 7/00 |
| | --- | | |
| P,X | JOURNAL OF THE AMERICAN CHEMICAL SOCIETY, Band 106, Nr. 12, 13. Juni 1984, Seiten 3695-3696, American Chemical Society, US; YI-FONG WANG et al.: "Bifunctional chiral synthons via biochemical methods. 3. Optical purity enhancement in enzymic asymmetric catalysis" | 1-3 | |
| | ----- | | |

Der vorliegende Recherchenbericht wurde für alle Patentansprüche erstellt.

| Recherchenort | Abschlußdatum der Recherche | Prüfer |
|---|---|---|
| DEN HAAG | 06-09-1985 | WRIGHT M.W. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82